(19) Europäisches Patentamt
European Patent Office
Office européen des brevets

(11) Veröffentlichungsnummer : **0 042 566**
**B1**

(12) **EUROPÄISCHE PATENTSCHRIFT**

(45) Veröffentlichungstag der Patentschrift : **05.09.84**

(21) Anmeldenummer : **81104601.0**

(22) Anmeldetag : **15.06.81**

(51) Int. Cl.³ : **C 07 D211/90**, C 07 D401/04, A 61 K 31/44// C07D495/10, C07D495/20

# (54) 4,4-Disubstituierte 1,4-Dihydropyridine, Verfahren zu ihrer Herstellung sowie ihre Verwendung in Arzneimitteln.

(30) Priorität : **25.06.80 DE 3023821**

(43) Veröffentlichungstag der Anmeldung : **30.12.81 Patentblatt 81/52**

(45) Bekanntmachung des Hinweises auf die Patenterteilung : **05.09.84 Patentblatt 84/36**

(84) Benannte Vertragsstaaten : **AT BE CH DE FR GB IT LI NL SE**

(56) Entgegenhaltungen :
**DE-A- 1 963 186**
**DE-A- 2 349 538**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Goldmann, Siegfried, Dr.**
**Kuckuckstrasse 41**
**D-5600 Wuppertal 2 (DE)**
Erfinder : **Bossert, Friedrich, Dr.**
**Claudiusweg 7**
**D-5600 Wuppertal 1 (DE)**
Erfinder : **Kazda, Stanislav, Dr.**
**Gellertweg 18**
**D-5600 Wuppertal 1 (DE)**
Erfinder : **Vater, Wulf, Dr.**
**Menchendahlerstrasse 23**
**D-5090 Leverkusen 3 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 042 566 B1

Jouve, 18, rue St-Denis, 75001 Paris, France

## Beschreibung

Die vorliegende Erfindung betrifft neue 4,4-disubstituierte 1,4-Dihydropyridine ein Verfahren zu ihrer Herstellung sowie ihre Verwendung in Arzneimitteln, insbesondere in kreislaufbeeinflussenden Mitteln.

Es ist bereits bekannt, daß 1,4-Dihydropyridin-Derivate, die in 4-Position nur einen Substituenten tragen, interessante pharmakologische Eigenschaften aufweisen und insbesondere als kreislaufbeeinflussende Mittel verwendet werden können (vgl. F. Bossert und W. Vater, Naturwissenschaften *58*, 578 (1971) und DT-OS-2 117 571). Solche 4-monosubstituierten 1,4-Dihydropyridine sind durch « Hantzsch-Synthese » leicht zugänglich (vgl. A. Hantzsch, Justus Lieb. Ann. Chem. *215*, 1 (1882)).

Ferner sind aus DE-A-1 963 186 bestimmte coronarwirksame 1,4-Dihydropyridine bekannt geworden, welche in 4-Position u. a. einen Arylsulfonsäurerest tragen können.

1,4-Dihydropyridin-3,5-dicarbonsäureester-derivate die in 4-Position zwei Substituenten tragen sind neu und nach bekannten Methoden bisher nicht herstellbar (vgl. B. Loev und K.M. Snader, J. Org. Chem. *30*, 1914 (1965)). Auch die Addition von metallorganischen Verbindungen an Pyridinderivaten läßt sich nur durchführen, wenn die 4-Position des Pyridins nicht bereits substituiert ist (vgl. R. Lukes und J. Kuthan, Collect, Czech, Chem. Comm. *26*, 1845 (1961) ; J. Palacek, K. Vondra und J. Kuthan, ibid. *34*, 2991 (1969) und J.F. Biellmann. H. J. Callot und M. P. Goeldner, Tetrahedron *26*, 4665 (1970).

Die vorliegende Erfindung betrifft neue 4,4-disubstituierte 1,4-Dihydropyridine der allgemeinen Formel (I)

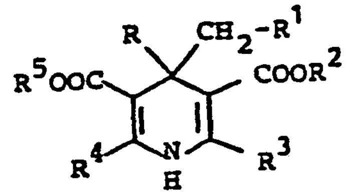

(I)

in welcher

R für Phenyl, Naphthyl, Furyl, Pyrryl oder Pyridyl steht, wobei diese Ringe gegebenenfalls 1, 2 oder 3 gleiche oder verschiedene Substituenten aus der Gruppe Phenyl, Alkyl, Alkenyl, Alkinyl, Alkoxy, Alkylen, Dioxyalkylen, Alkylamino mit jeweils bis zu 4 Kohlenstoffatomen, Halogen, Trifluormethyl, Trifluormethoxy, Cyano, Azido oder Carbonamido enthalten,

$R^3$ und $R^4$ gleich oder verschieden sind und jeweils für Wasserstoff oder einen Alkylrest mit bis zu 4 Kohlenstoffatomen, einen Phenylrest oder einen Benzylrest stehen,

$R^2$ und $R^5$ gleich oder verschieden sind und jeweils für einen geradkettigen, verzweigten oder cyclischen gesättigten oder ungesättigten Kohlenwasserstoffrest mit bis zu 6 Kohlenstoffatomen stehen, der durch Sauerstoff in der Kette unterbrochen sein kann und der gegebenenfalls substituiert ist durch Halogen, Pyridyl, Phenyl oder Phenoxy, wobei die Phenylgruppen ihrerseits substituiert sein können durch Halogen, Cyano oder Trifluormethyl,

$R^1$ für Wasserstoff, einen geradkettigen, verzweigten oder cyclischen gesättigten oder ungesättigten aliphatischen Kohlenwasserstoffrest mit bis zu 8 Kohlenstoffatomen steht, der gegebenenfalls in der Kette unterbrochen ist und der gegebenenfalls substituiert ist durch Phenyl, Phenoxy, Pyridyl oder Amino, wobei die Aminogruppe gegebenenfalls substituiert ist durch zwei gleiche oder verschiedene Substituenten aus der Gruppe Alkyl, Alkoxyalkyl mit jeweils bis zu 4 Kohlenstoffatomen, Phenyl oder Benzyl, und wobei der Phenyl- und der Phenoxyrest gegebenenfalls substituiert sind durch Halogen, Cyano, Trifluormethyl, Alkyl, Alkoxy oder Alkylamino mit jeweils 1 bis 4 Kohlenstoffatomen und

n für 0, 1 oder 2 steht,

sowie ihre pharmazeutisch unbedenklichen Säureadditionssalze.

Die Erfindung betrifft weiterhin ein Verfahren zur Herstellung von Verbindungen der allgemeinen Formel (I), welches dadurch gekennzeichnet ist, daß man 4,4-disubstituierte Spiro-1,4-dihydropyridine der allgemein Formel (II)

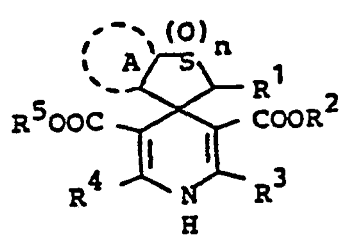

(II)

in welcher

$R^1$, $R^2$, $R^3$, $R^4$ und $R^5$ die oben angegebene Bedeutung haben,

(A) die gleiche Bedeutung wie der Substituent R besitzt und

n 0, 1 oder 2 bedeutet,

in an sich bekannter Weise in Gegenwart von Schwermetallkatalysatoren und gegebenenfalls in Gegenwart von inerten organischen Lösungsmitteln bei Temperaturen zwischen 0 und 120 °C entschwefelt und die so erhaltenen schwefelfreien erfindungsgemäßen Verbindungen in üblicher Weise isoliert.

Besonders vorteilhaft ist die Verwendung von Raney-Nickel als Schwermetallkatalysator.

Die Entschwefelung erfolgt nach an sich bekannten Methoden (vgl. Org. React. *12*, 356 bis 529 (1962) ; und Chem. Rev. *62*, 347-404 (1962)).

Die als Ausgangsstoffe verwendeten 4,4-disubstituierten Spiro-1,4-dihydropyridine der allgemeinen Formel (II) sind bisher noch nicht bekannt, können jedoch nach bekannten Methoden hergestellt werden. Ein möbliches Herstellungsverfahren geht von bekannten 1,4-Dihydropyridinen aus (vgl. DT-OS-2 117-571), welche zu entsprechenden Pyridinverbindungen oxidiert werden, z. B. durch Oxidation mit Chloranil (vgl. J. Am. Chem. Soc. *79*, 3477 (1957)). Diese Pyridinverbindungen, welche in 4-Position einen Aryl- oder Heteroarylrest tragen, welcher seinerseits in Orthostellung einen Thio-, Sulfinyl- oder Sulfonylrest enthält, bei tiefen Temperaturen, vorzugsweise bei Temperaturen zwischen − 120 °C und + 30 °C in Gegenwart von Basen wie Alkali- und Erdalkalihydriden und Alkoholaten oder Amiden, gegebenenfalls in Gegenwart von inerten organischen Lösungsmitteln, zu den entsprechenden Spiroverbindungen cyclisiert.

Die Verbindungen der allgemeinen Formel (I) zeigen interessante biologische Wirkungen. Sie besitzen insbesondere ein breites und vielseitiges pharmakologisches Wirkungsspektrum. Im einzelnen seien folgende Hauptwirkungen genannt :

1. Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen nitritähnlichen herzentlastenden Effekt verstärkt.

Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3. Der Tonus der glatten Muskulatur der Gefäße wird unter der Wirkung der Verbindungen stark vermindert. Diese gefäßspasmolytische Wirkung kann im gesamten Gefäßsystem staffinden, oder sich mehr oder weniger isoliert in umschriebenen Gefäßgebieten (wie z. B. dem Zentralnervensystem) manifestieren.

4. Die Verbindungen senken den Blutdruck von normotonen und hypertonen Tieren und können somit als antihypertensive Mittel verwendet werden.

5. Die Verbindungen haben stark muskulär-spasmolytische Wirkungen, diese an der glatten Muskulatur des Magens, Darmtraktes, des Urogenitaltraktes und des Respirationssystems deutlich werden.

Die neuen Wirkstoffe können in bekannter Weise in die üblichen Formulierung übergeführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt :

Wasser, nicht-toxische organische Lösungsmittel, wie Paraffine (z. B. Erdölfraktionen), pflanzliche Öle (z. B. Erdnuß-/Sesamöl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Roh-, Milch- und Traubenzucker), Emulgiermittel (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixiere, die für orale Anwendungen gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg Körpergewicht pro Tag zur Erzielung wirksamer

Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit von Körpergewicht des Versuchstieres bzw. der Art des Applikationsweges, aber auch aufgrund der Tierart und deren individullem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Midestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Von besonderem Interesse sind Verbindungen der allgemeinen Formel (I), in welcher

R für Phenyl, Furyl, Pyrryl oder Pyridyl steht, wobei die genannten Ringe gegebenenfalls 1 oder 2 gleiche oder verschiedene Substituenten aus der Gruppe Phenyl, Alkyl, Alkoxy, Alkylamino, jeweils 1 bis 4 Kohlenstoffatomen in den Alkyl- und Alkoxyresten, Halogen, Trifluormethyl, Trifluormethoxy, Cyano oder Azido enthalten,

$R^3$ und $R^4$ gleich oder verschieden sind und jeweils für Wasserstoff, Alkyl mit 1 bis 4 Kohlenstoffatomen, Phenyl oder Benzyl stehen,

$R^2$ und $R^5$ gleich oder verschieden sind und jeweils für einen Alkylrest mit 1 bis 6 Kohlenstoffatomen stehen, der gegebenenfalls durch Sauerstoff in der Kette unterbrochen ist oder der gegebenenfalls durch Halogen oder Phenyl substituiert ist,

$R^1$ für Alkyl oder Alkinyl mit bis zu 6 Kohlenstoffatomen steht, wobei der Alkylrest gegebenenfalls durch einen Sauerstoff in der Kette unterbrochen ist und gegebenenfalls substituiert ist durch Phenyl, Phenoxy oder Amino und wobei die Aminogruppe gegebenenfalls substituiert ist durch zwei gleiche oder verschiedene Substituenten aus der Gruppe Alkyl mit 1 bis 4 Kohlenstoffatomen, Phenyl oder Benzyl und

n für 0 und 1 steht.

Bei Kenntnis des Standes der Technik konnte nicht erwartet werden, daß die neuen erfindungsgemäßen Verbindungen, die sich durch ihre Zweifachsubstitution in 4-Stellung von allen bekannten Dihydropyridinen eindeutig unterscheiden, so vorteilhafte pharmakologische Wirkungen besitzen. Für diese neuen Stoffe ist erfahrungsgemäß mit einem anderen Wirkungsmechanismus zu rechnen, so daß die erfindungsgemäßen Verbindungen auch in solchen Fällen angewandt werden können, in denen bereits bekannte Arzneimittel Unverträglichkeiten oder Gewöhnung zeigen. Sie Stellen somit eine Bereicherung der Pharmazie dar.

Die nachfolgenden Beispiele erläutern beispielhaft die Herstellung einiger erfindungsgemäßer Verbindungen.

## Herstellung von Ausgangsverbindungen

### Beispiel A

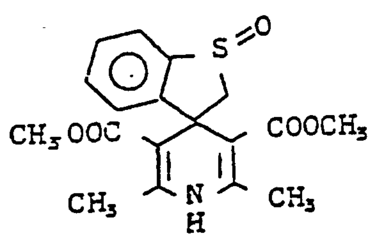

150 mmol 2,6-Dimethyl-4-(2-methylsulfinyl-phenyl)-pyridin-3,5-dicarbonsäure-dimethylester werden in 500 ml wasserfreiem Tetrahydrofuran gelöst und bei − 78 °C mit 300 mmol Lithiumdiisopropylamid versetzt. Unmittelbar darauf werden 50 ml Methanol, festes Ammoniumchlorid und 1 l Wasser zugesetzt. Der Niederschlag wird abgesaugt, in Wasser gewaschen und bei 100 °C getrocknet. Man erhält 4,3′-Spiro [2,6-dimethyl-3,5-bismethoxycarbonyl-1,4-dihydropyridin]-[2′,3′-dihydro-1′-benzothiophen-1′-oxid] vom Fp. : 286-289 °C (Zers.).

Ausbeute : 80 % der Theorie.

### Beispiel B

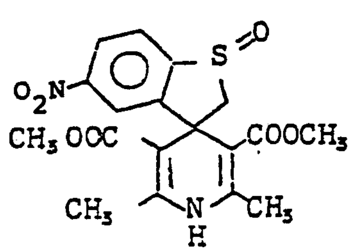

135 mmol 2,6-Dimethyl-4-(2-methylsulfinyl-5-nitrophenyl)-pyridin-3,5-dicarbonsäuredimethylester werden in Diethylether gelöst und bei 20 °C mit 270 mmol Kaliumhydrid umgesetzt und protoniert. Das Lösungsmittel wird abrotiert, in Methylenchlorid aufgenommen, getrocknet und erneut einrotiert. Der Rückstand kristallisiert beim Verreiben mit Essigester.

Man erhält 4,3'-Spiro [2,6-dimethyl-3,5-bismethoxycarbonyl-1,4-dihydropyridin]-[5'-nitro-2',3'-dihydro-1'-benzothiophen-1'-oxid] vom Fp. : 257-259 °C (Zers.).

Ausbeute : 33 % der Theorie.

Beispiel C

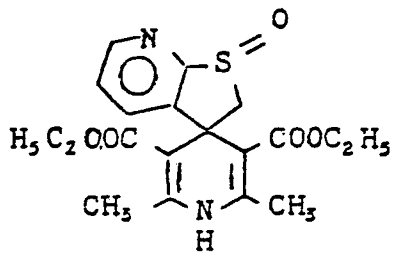

21 mmol 2,3-Dimethyl-4-(2-methylsulfinylpyridyl-3)-pyridin-3,5-dicarbonsäurediethylester werden in Dioxan gelöst und analog Beispiel 1 mit Lithiumdiethylamid umgesetzt und aufgearbeitet.

Man erhält 4,3'-Spiro [2,6-dimethyl-3,5-bisethoxycarbonyl-1,4-dihydropyridin]-[thieno-[2,3,6] pyridin-2',3'-dihydro-1'-oxid] vom Fp. : 253-256 °C.

Ausbeute : 27 % der Theorie.

Beispiel D

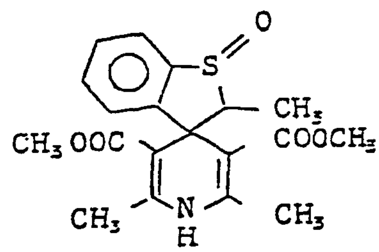

125 mmol 2,6-Dimethyl-4-(2-ethylsulfinylphenyl)-pyridin-3,5-dicarbonsäuredimethylester werden analog Beispiel 1 mit Lithiumdiisopropylamid bei – 100 °C umgesetzt und protoniert. Das Lösungsmittel wird abrotiert, der Rückstand mit Methylenchlorid extrahiert, über $Na_2SO_4$ getrocknet und einrotiert. Der Rückstand wird in Aceton gelöst und mit Ether gefällt. Man erhält 4,4'-Spiro [2,6-dimethyl-3,5-bismethoxycarbonyl-1,4-dihydropyridin]-[2'-methyl-2',3'-dihydro-1'-benzothiophen-1'-oxid] als Diastereomerengemisch mit dem Fp. : 200-206 °C.

Ausbeute : 41 % der Theorie.

Erfindungsgemäße Verbindungen

Beispiel 1

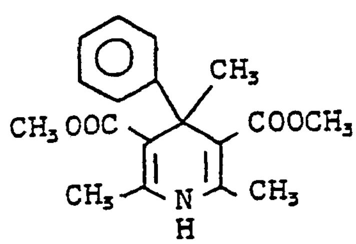

12 g (33 mmol) 4,3'-Spiro [2,6-dimethyl-3,5-bismethoxycarbonyl-1,4-dihydropyridin]-[2',3'-dihydro-1'-benzothiophen-1'-oxid] (Beispiel A) wurden in 600 ml 75 %igem wäßrigem Ethanol gelöst und zusammen mit 100 g Raney-Nickel (in $H_2O$ suspendiert) 5 Stunden am Rückfluß gekocht. Nach Abkühlung wurde abgenutscht, einrotiert und aus Essigester umkristallisiert. Man erhielt 74 % 2,6-Dimethyl-4-phenyl-4-methyl-1,4-dihydropyridin-3,5-dicarbonsäure-dimethylester mit dem Fp. : 148 bis 150 °C.

Beispiel 2

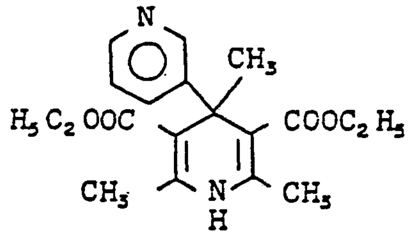

2,0 g (5,8 mmol) 4,3'-Spiro [2,6-dimethyl-3,5-bisethoxycarbonyl-1,4-dihydropyridin]-[thieno [2,3-b] pyridin-2',3'-dihydro-1'-oxid] (Beispiel C) wurden in 150 ml wäßrigem Ethanol gelöst und mit 20 g Raney-Nickel (in $H_2O$ suspendiert) 45 Minuten am Rückfluß gekocht und nach Abkühlung abgesaugt. Das Filtrat wurde einrotiert. Man erhielt 68 % 2,6-Dimethyl-4-(3-pyridyl)-4-methyl-1,4-dihydropyridin-3,5-dicarbonsäurediethylester mit dem Fp. 164 bis 167 °C.

Beispiel 3

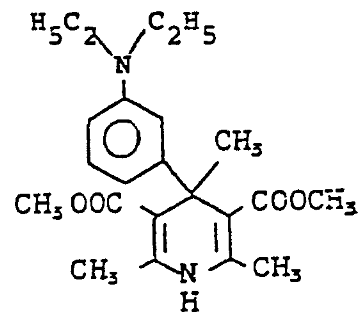

5,0 g (12 mmol) 4,3'-Spiro [2,6-dimethyl-3,5-bismethoxycarbonyl-1,4-dihydropyridin]-[5'-nitro-2',3'-dihydro-1'-benzothiophen-1'-oxid] (Beispiel B) wurden in 300 ml 75 %igem wäßrigem Ethanol gelöst und mit 50 g Raney-Nickel (in $H_2O$ suspendiert) 5 Stunden am Rückfluß gekocht. Es wurde abgesaugt, einrotiert, das verbleibende Öl in wenig Ether gelöst und mit Petrolether/60-70° gefällt. Man erhielt 25 % 2,3-Dimethyl-4-(3-diethylaminophenyl)-4-methyl-1,4-dihydropyridin-3,5-dicarbonsäuredimethylester mit dem Fp. : 103-105 °C.

Beispiel 4

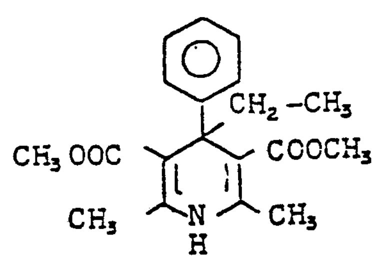

5,0 g (13 mmol) 4,3'-Spiro [2,6-dimethyl-3,5-bismethoxycarbonyl-1,4-dihydropyridin]-[2'-methyl-2',3'-dihydro-1'-benzothiophen-1'-oxid] (Beispiel D) wurden in 150 ml 75 %igem wäßrigem Ethanol gelöst und mit 50 g Raney-Nickel 5 Stunden am Rückfluß gekocht. Danach Zugabe von weiteren 20 g Raney-Nickel und weitere 6 Stunden Rückfluß.

Nach Absaugen des Nickels wurde das Filtrat einrotiert und der Rückstand an Kieselgel mit Ether chromatographiert ($R_F = 0.52$). Das Eluat wurde einrotiert, in wenig Ether gelöst und mit Petrolether/60-70 °C ausgefällt. Man erhielt 11 % 2,6-Dimethyl-4-phenyl-4-ethyl-1,4-dihydropyridin-3,5-dicarbonsäuredimethylester mit dem Fp. : 107 bis 109 °C.

**Ansprüche**

1. 4,4-disubstituierte 1,4-Dihydropyridine der allgemeinen Formel (I)

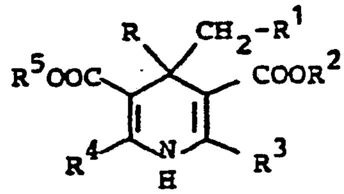

(I)

in welcher

R für Phenyl, Naphthyl, Furyl, Pyrryl oder Pyridyl steht, wobei diese Ringe gegebenenfalls 1, 2 oder 3 gleiche oder verschiedene Substituenten aus der Gruppe Phenyl, Alkyl, Alkenyl, Alkinyl, Alkoxy, Alkylen, Dioxyalkylen, Alkylamino mit jeweils bis zu 4 Kohlenstoffatomen, Halogen, Trifluormethyl, Trifluormethoxy, Cyano, Azido oder Carbonamido enthalten,

$R^3$ und $R^4$ gleich oder verschieden sind und jeweils für Wasserstoff oder einen Alkylrest mit bis zu 4 Kohlenstoffatomen, einen Phenylrest oder einen Benzylrest stehen,

$R^2$ und $R^5$ gleich oder verschieden sind und jeweils für einen geradkettigen, verzweigten oder cyclischen gesättigten oder ungesättigten Kohlenwasserstoffrest mit bis zu 6 Kohlenstoffatomen stehen, der durch Sauerstoff in der Kette unterbrochen sein kann und der gegebenenfalls substituiert ist durch Halogen, Pyridyl, Phenyl oder Phenoxy, wobei die Phenylgruppen ihrerseits substituiert sein können durch Halogen, Cyano oder Trifluormethyl,

$R^1$ für Wasserstoff, einen geradkettigen, verzweigten oder cyclischen gesättigten oder ungesättigten aliphatischen Kohlenwasserstoffrest mit bis zu 8 Kohlenstoffatomen steht, der gegebenenfalls in der Kette unterbrochen ist und der gegebenenfalls substituiert ist durch Phenyl, Phenoxy, Pyridyl oder Amino, wobei die Aminogruppe gegebenenfalls substituiert ist durch zwei gleiche oder verschiedene Substituenten aus der Gruppe Alkyl, Alkoxyalkyl mit jeweils bis zu 4 Kohlenstoffatomen, Phenyl oder Benzyl, und wobei der Phenyl- und der Phenoxyrest gegebenenfalls substituiert sind durch Halogen, Cyano, Trifluormethyl, Alkyl, Alkoxy oder Alkylamino mit jeweils 1 bis 4 Kohlenstoffatomen und

n für 0, 1 oder 2 steht,

sowie ihre pharmazeutisch unbedenklichen Säureadditionssalze.

2. Verbindungen der allgemeinen Formel (I) gemäß Anspruch 1, in welcher

R für Phenyl, Furyl, Pyrryl oder Pyridyl steht, wobei die genannten Ringe gegebenenfalls 1 oder 2 gleiche oder verschiedene Substituenten aus der Gruppe Phenyl, Alkyl, Alkoxy, Alkylamino, jeweils 1 bis 4 Kohlenstoffatomen in den Alkyl- und Alkoxyresten, Halogen, Trifluormethyl, Trifluormethoxy, Cyano oder Azido enthalten,

$R^3$ und $R^4$ gleich oder verschieden sind und jeweils für Wasserstoff, Alkyl mit 1 bis 4 Kohlenstoffatomen, Phenyl oder Benzyl stehen,

$R^2$ und $R^5$ gleich oder verschieden sind und jeweils für einen Alkylrest mit 1 bis 6 Kohlenstoffatomen stehen, der gegebenenfalls durch Sauerstoff in der Kette unterbrochen ist oder der gegebenenfalls durch Halogen oder Phenyl substituiert ist,

$R^1$ für Wasserstoff, Alkyl oder Alkinyl mit bis zu 6 Kohlenstoffatomen steht, wobei der Alkylrest gegebenenfalls durch einen Sauerstoff in der Kette unterbrochen ist und gegebenenfalls substituiert ist durch Phenyl, Phenoxy oder Amino und wobei die Aminogruppe gegebenenfalls substituiert ist durch zwei gleiche oder verschiedene Substituenten aus der Gruppe Alkyl mit 1 bis 4 Kohlenstoffatomen, Phenyl oder Benzyl und

n für 0 oder 1 steht.

3. Verfahren zur Herstellung von Verbindungen der allgemeinen Formel (I) gemäß Anspruch 1 und 2 dadurch gekennzeichnet, daß man 4,4-disubstituierte Spiro-1,4-dihydropyridine der allgemeinen Formel (II)

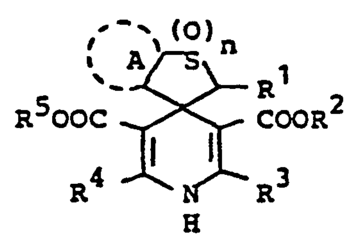

(II)

in welcher

$R^1$, $R^2$, $R^3$, $R^4$ und $R^5$ die in den Ansprüchen 1 und 2 angegebene Bedeutung haben,

A die gleiche Bedeutung wie der Substituent R aus Anspruch 1 oder 2 besitzt und

n 0, 1 oder 2 bedeutet,

in an sich bekannter Weise in Gegenwart von Schwermetallkatalysatoren und gegebenenfalls in Gegenwart von inerten organischen Lösungsmitteln bei Temperaturen zwischen 0 und 120 °C entschwefelt und die so erhaltenen schwefelfreien erfindungsgemäßen Verbindungen in üblicher Weise isoliert.

4. Arzneimittel, enthaltend mindestens eine Verbindung der allgemeinen Formel (I) gemäß Ansprüchen 1 und 2.

5. Verbindungen der allgemeinen Formel (I) gemäß Ansprüchen 1 und 2 zur Verwenaung bei der Bekämpfung von Kreislauferkrankungen.

6. Verfahren zur Herstellung von Arzneimitteln, dadurch gekennzeichnet, daß man Verbindungen der allgemeinen Formel (I) gemäß Ansprüchen 1 und 2 gegebenenfalls unter Verwendung üblicher Hilfs- und Trägerstoffe in eine geeignete Applikationsform überführt.

7. Verfahren gemäß Anspruch 3, dadurch gekennzeichnet, daß man Raney-Nickel als Schwermetallkatalysatoren verwendet.

**Claims**

1. 4,4-Disubstituted 1,4-dihydropyridines of the general formula (I)

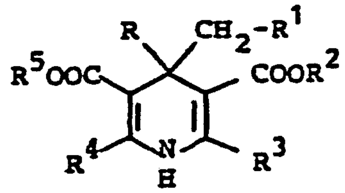

(I)

in which

R represents phenyl, naphthyl, furyl, pyrryl or pyridyl, these rings optionally containing 1, 2 or 3 identical or different substituents from the group comprising phenyl, alkyl, alkenyl, alkinyl, alkoxy, alkylene, dioxyalkylene and alkylamino with in each case up to 4 carbon atoms, halogen, trifluoromethyl, trifluoromethoxy, cyano, azido and carboxamido,

$R^3$ and $R^4$ are identical or different and each represent hydrogen or an alkyl radical with up to 4 carbon atoms, a phenyl radical or a benzyl radical,

$R^2$ and $R^5$ are identical or different and each represent a straight-chain, branched or cyclic, saturated or unsaturated hydrocarbon radical which has up to 6 carbon atoms, can be interrupted in the chain by oxygen and is optionally substituted by halogen, pyridyl, phenyl or phenoxy, it being possible for the phenyl groups in turn to be substituted by halogen, cyano or tirfluoromethyl,

$R^1$ represents hydrogen, or a straight-chain, branched or cyclic, saturated or unsaturated aliphatic hydrocarbon radical which has up to 8 carbon atoms, is optionally interrupted in the chain and is optionally substituted by phenyl, phenoxy, pyridyl or amino, the amino group optionally being substituted by two identical or different substituents from the group comprising alkyl, alkoxyalkyl with in each case up to 4 carbon atoms, phenyl and benzyl, and the phenyl and the phenoxy radical optionally being substituted by halogen, cyano, trifluoromethyl, alkyl, alkoxy or alkylamino with in each case 1 to 4 carbon atoms and

n represents 0, 1 or 2,

and pharmaceutically acceptable acid addition salts thereof.

2. Compounds of the general formula (I) according to Claim 1, in which

R represents phenyl, furyl, pyrryl or pyridyl, the indicated rings optionally containing 1 or 2 identical or different substituents from the group comprising phenyl, alkyl, alkoxy, alkylamino, in each case 1 to 4 carbon atoms in the alkyl and alkoxy radicals, halogen, trifluoromethyl, trifluoromethoxy, cyano and azido,

$R^3$ and $R^4$ are identical or different and each represent hydrogen, alkyl with 1 to 4 carbon atoms, phenyl or benzyl,

$R^2$ and $R^5$ are identical or different and each represent an alkyl radical which has 1 to 6 carbon atoms and is optionally interrupted in the chain by oxygen or is optionally substituted by halogen or phenyl,

$R^1$ represents hydrogen, alkyl or alkinyl with up to 6 carbon atoms, the alkyl radical optionally being interrupted in the chain by one oxygen and optionally being substituted by phenyl, phenoxy or amino, and the amino group optionally being substituted by two identical or different substituents from the group comprising alkyl with 1 to 4 carbon atoms, phenyl and benzyl and

n represents 0 or 1.

3. Process for the preparation of compounds of the general formula (I) according to Claim 1 and 2, characterised in that 4,4-disubstituted spiro-1,4-dihydropyridines of the general formula (II)

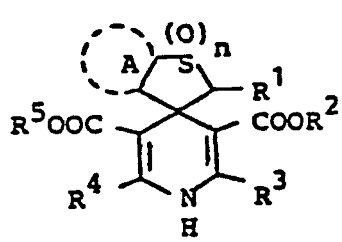

(II)

in which

$R^1$, $R^2$, $R^3$, $R^4$ and $R^5$ have the meaning indicated in Claims 1 and 2,
A has the same meaning as the substituent R in Claim 1 or 2 and
n denotes 0, 1 or 2,

are desulphurised in a manner known per se in the presence of heavy metal catalysts and if appropriate in the presence of inert organic solvents at temperatures between 0 and 120 °C and the sulphur-free compounds according to the invention thus obtained are isolated in a customary manner.

4. Medicaments containing at least one compound of the general formula (I) according to Claims 1 and 2.

5. Compounds of the general formula (I) according to Claims 1 and 2 for use in combating circulatory illnesses.

6. Process for the preparation of medicaments, characterised in that compounds of the general formula (I) according to Claims 1 and 2 are converted into a suitable form of administration if appropriate using customary auxiliaries and excipients.

7. Process according to claim 3, characterised in that Raney-Nickel is used as the heavy metal catalyst.

**Revendications**

1. 1,4-dihydropyridines 4,4-disubstituées de formule générale (I)

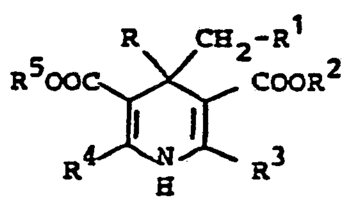

(I)

dans laquelle

R est un groupe phényle, naphtyle, furyle, pyrryle ou pyridyle, ces noyaux contenant éventuellement 1, 2 ou 3 substituants égaux ou différents du groupe phényle, alkyle, alcényle, alcynyle, alkoxy, alkylène, dioxyalkylène, alkylamino ayant dans chaque cas jusqu'à 4 atomes de carbone, halogéno, trifluorométhyle, trifluorométhoxy, cyano, azido ou carboxamido,

$R^3$ et $R^4$ sont égaux ou différents et représentent chacun l'hydrogène ou un reste alkyle ayant jusqu'à 4 atomes de carbone, un reste phényle ou un reste benzyle,

$R^2$ et $R^5$ sont égaux ou différents et représentent chacun un reste d'hydrocarbure saturé ou insaturé à chaîne droite, ramifié ou cyclique ayant jusqu'à 6 atomes de carbone, qui peut être interrompu dans la chaîne par de l'oxygène et qui est substitué le cas échéant par un halogène, un groupe pyridyle, phényle ou phénoxy, les groupes phényle pouvant être substitués quant à eux par un halogène, un groupe cyano ou un groupe trifluorométhyle,

$R^1$ est l'hydrogène, un reste d'hydrocarbure aliphatique saturé ou insaturé à chaîne droite, ramifié ou cyclique ayant jusqu'à 8 atomes de carbone, qui est éventuellement interrompu dans la chaîne et qui est substitué le cas échéant par un groupe phényle, phénoxy, pyridyle ou amino, le groupe amino étant éventuellement substitué par deux substituants égaux ou différents du groupe des substituants alkyle, alkoxyalkyle ayant chacun jusqu'à 4 atomes de carbone, phényle ou benzyle, et le reste phényle et le reste phénoxy étant éventuellement substitués par des substituants halogéno, cyano, trifluorométhyle, alkyle, alkoxy ou alkylamino ayant chaun 1 à 4 atomes de carbone, et

n est égal à 0, 1 ou 2,

ainsi que leurs sels d'addition d'acides pharmaceutiquement acceptables.

2. Composés de formule (I) suivant la revendication 1, dans laquelle

R représente un groupe phényle, furyle, pyrryle, ou pyridyle, les noyaux mentionnés contenant éventuellement 1 ou 2 substituants égaux ou différents choisis dans le groupe des substituants phényle, alkyle, alkoxy, alkylamino ayant chacun jusqu'à 4 atomes de carbone dans les restes alkyle et alkoxy, halogéno, trifluorométhyle, trifluorométhoxy, cyano ou azido,

$R^3$ et $R^4$ sont égaux ou différents et représentent chacun l'hydrogène ou un substituant alkyle ayant 1 à 4 atomes de carbone, phényle ou benzyle,

$R^2$ et $R^5$ sont égaux ou différents et représentent chacun un reste alkyle ayant 1 à 6 atomes de carbone, qui est éventuellement interrompu dans la chaîne par de l'oxygène ou qui est substitué le cas échéant par un halogène ou un groupe phényle,

$R^1$ représente l'hydrogène, un groupe alkyle ou alcynyle ayant jusqu'à 6 atomes de carbone, le reste alkyle étant éventuellement interrompu dans la chaîne par de l'oxygène et étant éventuellement substitué par un substituant phényle, phénoxy ou amino et le groupe amino étant éventuellement substitué par

deux substituants égaux ou différents du groupe des substituants alkyle ayant 1 à 4 atomes de carbone, phényle ou benzyle et

n est égal à 0 ou à 1.

3. Procédé de production de composés de formule générale (I) suivant les revendications 1 et 2, caractérisé en ce qu'on désulfure des spiro-1,4-dihydropyridines 4,4-disubstituées de formule générale (II)

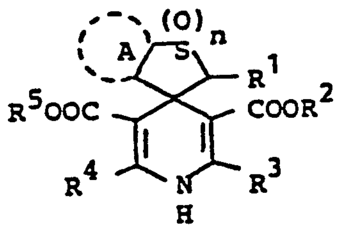

(II)

dans laquelle

$R^1$, $R^2$, $R^3$, $R^4$ et $R^5$ ont les définitions indiquées dans les revendications 1 et 2,

A a la même définition que celle qui a été donnée pour le substituant R dans la revendication 1 ou 2 et

n est égal à 0, 1 ou 2,

d'une manière connue en présence de catalyseurs à base de métaux lourds et, le cas échéant, en présence de solvants organiques inertes, à des températures comprises entre 0 et 120 °C et on isole d'une manière classique les composés désulfurés de l'invention ainsi obtenus.

4. Médicament contenant au moins un composé de formule générale (I) suivant les revendications 1 et 2.

5. Composés de formule générale (I) suivant les revendications 1 et 2, destinés à être utilisés dans la lutte contre des maladies de la circulation.

6. Procédé de préparation de médicaments, caractérisé en ce qu'on convertit en une forme d'application appropriée des composés de formule générale (I) suivant les revendications 1 et 2, éventuellement en utilisant des adjuvants et des supports classiques

7. Procédé suivant la revendication 3, caractérisé en ce qu'on utilise le nickel de Raney comme catalyseur à base de métal lourd.